# EUROPEAN PATENT APPLICATION

(11) **EP 3 372 559 A1**
(43) Date of publication of application: **12.09.2018**
(21) Application number: 16861655.5
(22) Date of filing: 04.11.2016
(51) Int. Cl.: C02F 3/00, C02F 101/30

(54) **METHOD AND SYSTEM FOR ELIMINATING MICROCONTAMINANTS BY MEANS OF A REACTOR WITH AN ENZYME IMMOBILISED IN MAGNETIC NANOPARTICLES AND AN INTERNAL-SEPARATION UNIT**

(30) Priority: 06.11.2015 ES 201531599
(71) Applicant: Universidade de Santiago de Compostela, 15782 Santiago de Compostela (a Coruña) (ES)
(72) Inventor: MOLDES DIZ, Yolanda, 15782 Santiago de Compostela (ES); EIBES GONZÁLEZ, Gemma, 15782 Santiago de Compostela (ES); ARCA RAMOS, Adriana, 15782 Santiago de Compostela (ES); VÁZQUEZ VÁZQUEZ, Carlos, 15782 Santiago de Compostela (ES); FONDADO FONDADO, Alfonso, 15782 Santiago de Compostela (ES); MIRA PÉREZ, Jorge, 15782 Santiago de Compostela (ES); LEMA RODICIO, Juan Manuel, 15782 Santiago de Compostela (ES); FEIJOO COSTA, Gumersindo, 15782 Santiago de Compostela (ES); MOREIRA VILAR, María Teresa, 15782 Santiago de Compostela (ES)
(74) Representative: Elzaburu S.L.P.
(86) International application number: PCT/ES2016/070778
(87) International publication number: WO 2017/077159

(57) **Abstract**

Method and system for eliminating microcontaminants by means of a reactor with an enzyme immobilised on magnetic nanoparticles and an internal-separation unit. The present invention relates to a method and system for eliminating organic microcontaminants present in effluents from wastewater treatment plants (WWTPs) or industrial effluents by means of peroxidase or laccase enzymes immobilised on magnetic nanoparticles by using an enzyme reactor with magnetic separation.

## Description

### TECHNICAL FIELD OF THE INVENTION

The present invention relates to a method and system for eliminating organic microcontaminants, such as industrial dyes and endocrine disrupting compounds present in effluents from wastewater treatment plants after secondary treatment, as well as in industrial effluents.

### STATE OF THE ART

The discharging of recalcitrant organic contaminants into aquatic environments significantly affects the viability of reusing water treated in wastewater treatment plants (WWTPs) due to the possible harmful effects these compounds have on living organisms, even in trace amounts (µg L⁻¹). Endocrine Disrupting Compounds (EDC), a group of substances that are naturally or anthropogenically produced with the capability of altering the functions of the endocrine system are of special concern, given that they can cause adverse effects to an organism and the progeny thereof. These compounds have been classified as emerging contaminants and have received special attention given that they are only partially eliminated in conventional WWTP processes, continuously being released into the environment (Liu Z. et al., 2009; Sci Total Environ 407: 731-748).

In effluents from textile or pulp and paper industries, the presence of organic microcontaminants in said effluents, such as synthetic dyes with a recalcitrant nature, is highly common, even after a conventional treatment system (Rani B. et al. 2014; Braz J Microb 45: 1055-1063).

In recent years different post-treatments for the elimination of organic microcontaminants based on advanced oxidation processes have been developed, such as ozonation, photodegradation, hypochlorite or chlorine oxides, photocatalysis, ultrasound, etc. However, in general these post-treatments are expensive, have poor specificity (Esplugas S. et al., 2002; Water Res 36: 1034-1042) and occasionally generate by-products that can potentially be more harmful than the original compound (Shapell N.W. et al., 2008; Environ Sci Technol 42: 1296-1300).

One alternative for the transformation of these microcontaminants into less toxic products is enzymatic treatment, which contributes to polymerisation and transformation in molecules without endocrine disruptive action and transforms them into products that are more easily degradable (Galliker P. et al., 2010; J Colloid Interface Sci 349: 98-105).

Oxidoreductase enzymes have been successfully applied for eliminating different EDCs, such as bisphenol A (BPA), nonylphenol, triclosan (Cabana H. et al., 2007; Eng Life Sci 7: 429-456) and synthetic dyes such as methyl green (MG), remazol brilliant blue B and reactive black (Kunamneni A. et al., 2008; Process Biochem 43: 169-178). There are mainly two types of oxidoreductases: peroxidases, such as lignin peroxidase (LiP), versatile peroxidase (VP), horseradish peroxidase (HRP) and manganese peroxidase (MnP) or oxidases such as laccase (Lac).

Peroxidases are hemoproteins that require the presence of hydrogen peroxide as an electron acceptor to carry out oxidation of substrates. They have oxidation potentials of up to 1.51 V. The LiP enzyme (EC 1.11.1.13) is characterised for its high redox potential which allows for oxidation of aromatic, phenolic and non-phenolic compounds, such as veratryl alcohol (Camarero S. et al., 1999; J Biol Chem 274: 10324-10330). In the case of MnP (EC 1.11.1.14), this enzyme oxidises Mn²⁺ to Mn³⁺, which acts as a diffusible agent, oxidising both phenolic units as well as non-phenolic units through the peroxidation of lipids (Wariishi H. et al., 1988; Biochemestry 27: 5365-5370). The VP enzyme (EC 1.11.1.16) is considered a hybrid between MnP and LiP, since it is capable of oxidising Mn²⁺ as well as non-phenolic compounds with high redox potential, such as veratryl alcohol (Wong D., 2009; Appl Biochem Biotech 157: 174-209). Lastly, the HRP enzyme (EC 1.11.1.7) allows for the oxidation of substrates such as phenolic compounds and aromatic amines (Ben-Pei W. et al., 2014; J Mol Catal B-Enzym 101: 101-107).

The capability of peroxidases for degrading microcontaminants has been demonstrated in several studies. Some noteworthy examples include Cheng W. et al., 2012; Enzyme Microb Tech 50: 204-208, in which phenol, natural hormones such as estrone (E1), estradiol (E2) and estriol (E3), and synthetic hormones such as ethinylestradiol (EE2) are degraded using the HRP enzyme. Taboada-Puig R. et al., 2011; Bioresour Technol 102: 6593-6599 used immobilised VP on CLEAs to eliminate bisphenol A, nonylphenol, triclosan and 17 α-estradiol.

Laccase (EC 1.10.3.2) is an enzyme with phenoloxidase activity which contains copper atoms in the active centre thereof and catalyses the oxidation of a wide variety of organic substances in a process coupled to the molecular oxygen reduction of water (Kunamneni A. et al., 2008; Process Biochem 43: 169-178). The wide specificity of substrate, the use of oxygen as an electron acceptor and the generation of water as a single by-product of the reaction (Bourbonnais R. et al., 1990; FEBS 1: 99-102) makes the enzyme highly applicable in different biotechnological processes. Despite they have a maximum redox potential of 0.8 V, lower than that of ligninolytic peroxidases, the presence of substrates with low molecular weight, known as mediators, allows for indirect oxidation of a wide range of phenolic and non-phenolic compounds (Cañas A. et al., 2010; Biotechnol Adv 28: 694-705).

The capability of laccase for eliminating microcontaminants has been demonstrated in a wide variety of studies. As noteworthy example can be found by Auriol M. et al., 2008; Chemosphere 70: 445-452, who used laccase from *Trametes versicolor for* eliminating the estrogenic activity associated with natural and synthetic hormones. Lloret L. et al., 2010; Bioch Eng Journal 51: 124-131 used laccase from *Myceliophthora thermophila* for eliminating different microcontaminants of the anti-inflammatory group: naproxen, diclofenac and different hormones. This same laccase was also successfully applied to degrade bisphenol A, nonylphenol and triclosan (Cabana H. et al., 2007; Chemosphere 67: 770-778).

However, for technical and economic reasons, in the majority of enzyme catalysed processes it is necessary to reuse or continually use biocatalysts, and as such, the use of free enzymes as a tertiary treatment is not viable (Katchalski-Katzir E. et al., 2000; J Mol Catal B-Enzym 10: 157-176). In this context, enzyme immobilisation can be considered as an alternative.

Enzyme immobilisation in solid supports provides several advantages over the use of free enzymes, since the separation of the immobilised enzyme from the reaction mixture by means of physical methods, such as filtration or sedimentation (centrifugation), is simpler and can be used in repeated cycles.

Among the supports, the use of magnetic nanoparticles provides several advantages, such as a high surface area, high enzyme charge, a reduction in problems of diffusion and a fast and easy recovery of the biocatalyst from the reaction means applying an external magnetic field. This way, the enzymes are subjected to a much less mechanical stress compared with centrifugation and sedimentation.

In several recent studies it has been shown that it is possible to immobilise enzymes on different types of magnetic nanoparticles with satisfactory results. As a noteworthy example, we may point out that Zimmermann Y.et al., 2011; Appl Microbiol Biot 92: 169-178 immobilised laccase *Coriolopsis polyzona* on silica-coated magnetic nanoparticles. Kalkan N. et al., 2012; J Appl Polym Sci 123: 707-716 used chitosan-coated magnetic nanoparticles to immobilise lacasse from *Trametes versicolor.* Immobilization of HRP on magnetic nanoparticles has also been demonstrated (Corgié S. et al., 2012; Adv Funct Mater 22: 1940-1951).

These results can be considered the starting point for the development of magnetic enzyme reactors. However, the available literature on configurations of magnetic reactors is very limited. Some alternatives are provided below.

In recent investigations magnetic enzyme reactors have been used, such as the case of Wang F. et al., 2012; Bioresource Technol 110: 120-124, which designed a magnetically stabilized fluidised bed reactor, or Duan X. et al., 2014; ChemPhysChem 15: 974-980 in which the reactor had a system of electromagnets where the reaction took place, both for the degradation of phenol in wastewater. A disadvantage of these two configurations is the necessity to connect the reactor to an electric power supply, and this has additional drawbacks (cost of electricity, high temperatures, etc.). Ardao I. et al., 2013; Proceedings of the 2nd European Symposium of Water Technology and Management, Belgium, also presented a configuration of a continuous reactor for eliminating microcontaminants wherein the separation system was a magnet in the outlet stream that collects the nanoparticles and by means of a system of valves the flow is reversed, bringing the nanoparticles back to the reactor.

### DESCRIPTION OF THE INVENTION

One aspect of the present invention relates to a method for eliminating organic microcontaminants present in WWTP effluents or industrial effluents by means of peroxidase or laccase enzymes immobilised on magnetic nanoparticles by using an enzyme reactor with magnetic separation.

The method for eliminating organic microcontaminants present in secondary effluents from wastewater treatment plants (WWTP) or industrial effluents comprises the operation of a sequencing batch reactor according to the following stages:
a) applying a pre-treatment that reduces the concentration of suspended solids and colloids in the effluent;
b) pumping the effluent towards a reaction vessel that contains the enzyme with magnetic supports that immobilise the enzyme;
c) the reaction of the effluent with the immobilised enzyme;
d) retaining the immobilised enzyme by applying an external magnetic field;
e) discharging the stream that is free of microcontaminants into the aquatic environment;
f) removing the external magnetic field; and
g) pumping a fresh effluent to the reaction vessel.

The pre-treatment reduces the concentration of suspended solids and colloids in the water to be treated, with the aim of maximising the enzymatic stability in the reactor. In a specific embodiment of the invention, the pre-treatment comprises making the effluent of the treatment plant circulate through sand filter with a grain size lower than 1 mm or using a microfiltration membrane to filter the effluent before introducing it in the enzyme reactor.

The enzyme covalently immobilised onto solid supports is in the reaction vessel. When a peroxidase enzyme is used, the activity of the enzyme is comprised in the range of 50-1000 U/L. The activity of the LiP enzyme is determined by the test described by Tien and Kirk (Tien M. and Kirk T.K., 1984; Proc Natl Acad Sci USA 81: 2280-2284), the measurement of MnP or VP activity carried out through the test described by Taboada-Puig et al. (Taboada-Puig R. et al., 2011; Biotechnol Prog 27: 668-676) and the measurement of HRP activity is done according to the method described by Bindhu et al. (Bindhu L. et al., 2002; J Appl Polym Sci 88: 1456-1464). If laccase is used, the enzymatic activity is comprised in the range of 100-1500 U/L, according to the measurement protocol detailed in the literature (Zimmermann Y. et al., 2011; Appl Microbiol Biotechnol 92: 169-178). The operating temperature in the reactor must be in the range of 10-40°C, preferably at 25°C and the pH must be comprised between 3-8, preferably at a pH of 4.5 in the case that the enzyme is a peroxidase-type enzyme and at pH between 6-7 in the case that the enzyme is a laccase-type enzyme.

In a specific embodiment the supports used for the immobilization of the enzyme are silica-coated magnetic nanoparticles with a size comprised between 4-40 nm, the silica layer being comprised in the range of 0.5-10 nm. The magnetic nanoparticles comprise magnetic iron oxide (magnetite or maghemite) with a diameter preferably comprised in the range of 3-20 nm and have superparamagnetic properties.

The reaction stage comprises stirring the effluent with the immobilised enzyme mixture during a pre-determined reaction time, at an interval of minutes or hours, depending on the recalcitrant character of the target compound and at a stirring speed range of 100 to 600 rpm.

In another aspect of the invention, in the reaction stage of the effluent with the enzyme, an enzymatic activity control stage is carried out, which comprises:
a) measuring enzymatic activity in the reaction vessel;
b) if the enzymatic activity in the vessel is less than a minimum value, regeneration of the nanoparticles is carried out for immobilising new enzyme.
The minimum value of enzymatic activity is 100 U/L when the enzyme is a laccase-type enzyme and 50 U/L when the enzyme is a peroxidase-type enzyme.

In a specific embodiment of the invention the regeneration of nanoparticles for immobilising new enzyme is done according to the procedure described in Zhao G. et al., 2011; J Phys chem 115: 6350-6359 for the subsequent immobilization of enzymes following the method indicated in Zimmermann Y.et al., 2011; Appl Microbiol Biotechnol 92: 169-178.

In the case of using a peroxidase enzyme, hydrogen peroxide must be added to the reactor at a speed comprised between 5-100 µmol/L·min; furthermore, if the enzyme used is MnP or VP, a dicarboxylic organic acid is continuously added at a flow rate comprised in the range of 1-100µmol/L·min. Furthermore, in the case of using a peroxidase enzyme, the necessary cofactor is introduced in the vessel to complete the catalytic cycle of the enzyme; in the case that the enzyme is an LiP-type enzyme, the cofactor comprises veratryl alcohol, which is added at a flow rate comprised in the range of 0.5-1000 µmol/L·min; in the case that the enzyme is a MnP- or VP-type enzyme, the cofactor comprises Mn²⁺ which is added at a flow rate comprised in the range of 0.5-100 µmol/L·min.

In the case that the enzyme used is laccase, the concentration of dissolved oxygen in the reactor is measured by a dissolved oxygen sensor.

In another aspect of the invention, after the reaction period, the immobilised enzyme is retained by an external magnetic field applied in the reaction vessel (reactor) such that the discharge of the treated stream free of nanoparticles is possible. It is necessary to take samples of said stream to determine the enzymatic activity (which must be null by being retained in the reactor) as well as the concentration of microcontaminants after enzymatic treatment in the reactor. In the case that the concentration of contaminants at the outlet of the reactor is greater than a fixed maximum value, the reaction time increases.

In another aspect, the invention relates to a system for eliminating organic microcontaminants present in secondary effluents in wastewater treatment plants (WWTP) or industrial effluents by means of peroxidases or laccase enzymes through the use of a sequential batch reactor based on a reactor with an enzyme immobilised on magnetic nanoparticles with a magnetic separation system coupled to the reactor in the enzyme recovery stage. The system for eliminating organic microcontaminants comprises:
a) a pre-treatment system that reduces the concentration of suspended solids and colloids in the effluent through a bed of sand with a grain size of less than 1 mm or through a microfiltration membrane;
b) a first pumping system;
c) a reaction vessel or reactor that comprises an enzyme solution with magnetic supports that immobilise the enzyme;
d) a stirring system;
e) an enzymatic activity control system for the solution contained in the reaction vessel;
f) a magnetic separation system;
g) a second pumping system; and
h) a system for measuring microcontaminants in the enzymatic reaction vessel and in the outlet of the reactor.
In the system for eliminating microcontaminants, the first pumping system pumps the effluent from the pre-treatment system to the reaction vessel.

In a specific embodiment the reaction vessel or reactor comprises a stirred-tank reactor. In a specific embodiment, the stirred tank comprises a stirring system with a four-blade stirrer, coated in Teflon to prevent problems of adsorption of nanoparticles and of compounds to be degraded.

In a specific embodiment the supports used for the immobilisation of the enzyme comprise silica-coated magnetic nanoparticles with a size comprised between 4-40 nm, the silica layer being comprised in the range of 0.5-10 nm. The magnetic nanoparticles comprise magnetic iron oxide (magnetite or maghemite) with a diameter preferably comprised in the range of 3-20 nm and have superparamagnetic properties.

The enzymatic activity control system comprises:
a) an enzymatic activity sensor; and
b) a system for the regeneration of supports and subsequent immobilisation of the enzyme.
The operating temperature in the reactor is comprised in the range of 10-40°C, preferably at 25°C and the pH must be comprised between 3-8, preferably pH of 4.5 in the case of peroxidase, and pH 6-7 in the case of laccase.

In the case of using the peroxidase enzyme, the system for eliminating microcontaminants further comprises a system for continuously adding hydrogen peroxide, which is added at a flow rate comprised in the range of 5-100 µmol/L·min. The system for eliminating microcontaminants further comprises a system for the continuous addition of dicarboxylic organic acid when the enzyme used is manganese peroxidase, which is added at a flow rate comprised in the range of 1-100 µmol/L·min. In the case that the enzyme used is a peroxidase enzyme, the system further comprises a system for adding necessary cofactors to complete the catalytic cycle; said adding system incorporating veratryl alcohol, when the enzyme used is LiP, at a flow rate comprised in the range of 0.5-1000 µmol/L·min; and Mn²⁺ if the enzyme used is an MnP- or VP-type enzyme, at a flow rate comprised in the range of 0.5-100 µmol/L·min.

In the case that the enzyme used is laccase, the reaction vessel further comprises a control system for dissolved oxygen in the enzyme-effluent mixture that comprises a sensor for measuring the concentration of dissolved oxygen.

In a specific embodiment of the invention, the external magnetic separation system comprises one or several magnetic rods formed by permanent magnets aligned and mounted with alternate polarity, such that the same poles of contiguous magnets are facing one another. In the vicinity of the poles, the magnetic field is not homogenous, and the particles are attracted to the most intense area of the filed, which are the areas of the poles.

In a specific embodiment, the magnetic separation system comprises toroidal magnets of neodymium-iron-boron in an annular form with axial polarisation held together by means of a non-magnetic lower rod with safety stops at the ends. In a specific embodiment, the toroidal magnets have an inner diameter of 6 mm, outer diameter of 15 mm and a height of 6 mm. The magnetic nanoparticles are retained on the outer wall of non-magnetic cylindrical sheath and with a sufficiently narrow wall inserted in the vessel and open to the outside, in which the magnetic rods are introduced. By removing the rods, the nanoparticles are released and dragged by the stirring system.

The second pumping system pumps the stream free of microcontaminants to the reactor outlet.

The system for measuring microcontaminants takes samples of effluents entering the reactor and of the stream exiting the same to determine the concentration of microcontaminants after enzymatic treatment in the reactor.

In another aspect, the invention relates to the use of the aforementioned method and system for eliminating organic microcontaminants present in secondary effluents from wastewater treatment plants (WWTP) or industrial effluents.

### BRIEF DESCRIPTION OF THE FIGURES

The detailed embodiments in the figures are represented by way of example and not by way of limitation:

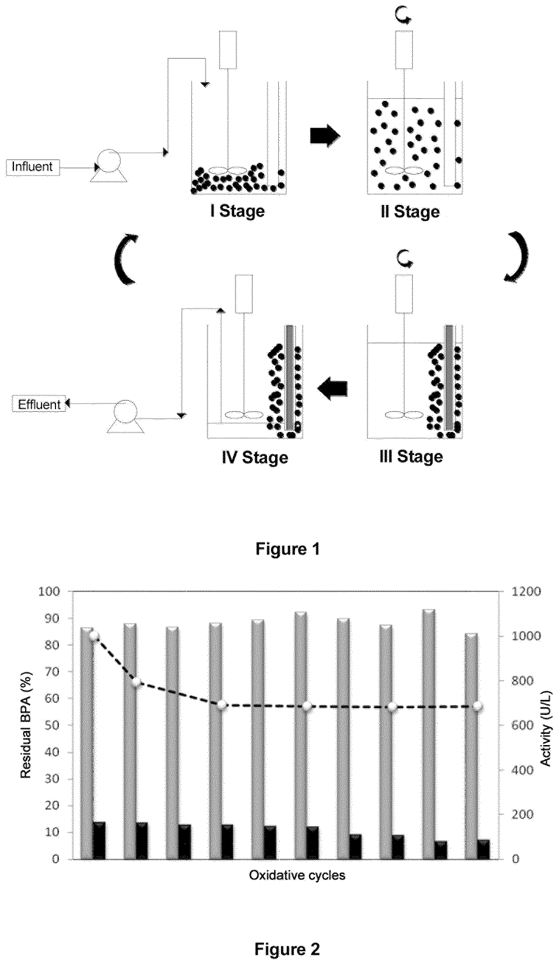

**Figure 1** shows a schematic diagram of the system of the sequential enzymatic reactor with a magnetic rod and the application thereof on microcontaminants. The influent enters the reactor with a specific concentration of microcontaminants, where the immobilised enzyme is located on magnetic nanoparticles in dissolution (Stage I). The reaction is left for the necessary operating time in each case (Stage II). After the reaction stage, the magnetic rod is introduced and the immobilised enzyme is retained on the magnetic nanoparticles by the magnetic field (Stage III). The outlet stream of the reactor is pumped and the effluent is obtained, which can discharge into the aquatic environment since it is free of microcontaminants (Stage IV). Lastly, the magnetic rod is removed and a new cycle is begun by adding the influent.
**Figure 2** Concentration profiles (percentage with respect to the concentration in the inlet stream) of BPA in the experiments of degradation (■) and in the control (■) in the outlet stream of the reactor and the activity of immobilised laccase on magnetic nanoparticles (-) in the sequential enzymatic reactor for treating BPA (100 µg/L) with immobilised laccase in 10 cycles with an operation time of 6 h.

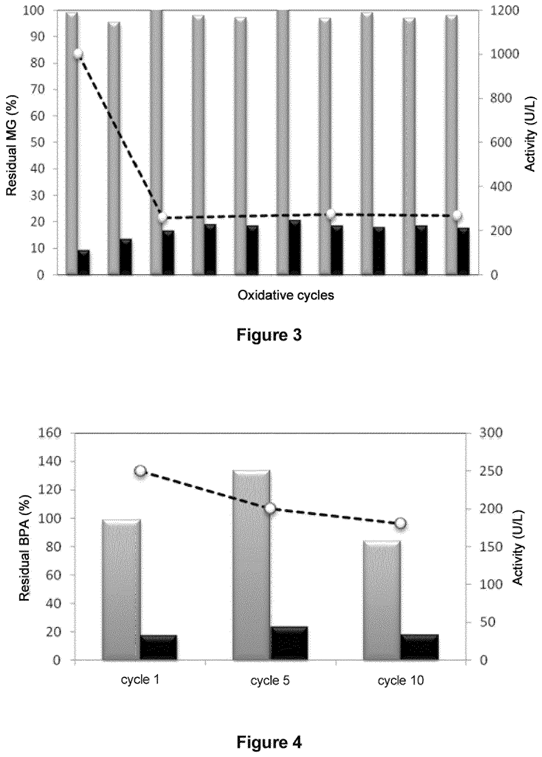

**Figure** 3 Concentration profiles (percentage with respect to the concentration in the inlet stream) of MG in the experiments of degradation (■) and in the control (■) in the outlet stream of the reactor and the activity of immobilised laccase in magnetic nanoparticles (-) in the sequential enzymatic reactor for treating methyl green dye (20 µg/L) with immobilised laccase in 10 cycles with an operation time of 24 h.
**Figure 4** Concentration profiles (percentage with respect to the concentration in the inlet stream) of BPA in the experiments of degradation (■) and in the control (■) in the outlet stream of the reactor and the activity of immobilised laccase on magnetic nanoparticles (-) in the sequential enzymatic reactor for treating BPA (100 µg/mL) with immobilised laccase in 10 cycles with an operation time of 6 h.

### EXAMPLES

### Example 1

The previously described method was applied for degrading bisphenol A (BPA) for wastewater treatment by means of immobilised laccase on magnetic nanoparticles in a sequencing batch reactor (SBR) with an operating volume of 20 mL coupled to a magnetic rod with the aim of retaining the enzyme and reusing it according to the diagram shown in Figure 1. The average enzyme concentration was 1000 U/L and the concentration of BPA in the influent was 100 µg/L. The operation conditions are detailed below: temperature 25°C; pH 6; stirring, 150 rpm; 10 cycles for 6 h each.

The elimination of BPA is determined by gas chromatography coupled to mass spectrometry. The enzyme activity was determined spectrophotometrically. The results show that the system eliminates approximately 90% throughout 10 cycles. (Figure 2)

### Example 2

The previously described method was applied for the discolouration of dyes in the degradation of MG by means of immobilised laccase on magnetic nanoparticles in a sequencing batch reactor (SBR) with an operating volume of 10 mL (Figure 1). The initial enzyme concentration was 1000 U/L and the concentration of MG was 20 mg/L. The operation conditions were as follows: temperature, 25°C; pH 5; stirring, 150 rpm; 7 cycles with an operation time of 24 h.

The discolouration of MG and the enzyme activity were determined spectrophotometrically. The results show that approximately 80% is eliminated throughout 10 cycles (Figure 3).

### Example 3

The previously described method was applied for the degradation of bisphenol A (BPA) for real wastewater treatment by means of immobilised laccase on magnetic nanoparticles in a sequencing batch reactor (SBR) with an operating volume of 500 mL coupled to a magnetic rod with the aim of retaining the enzyme and reusing it according to the diagram shown in Figure 1. It is a test with an increased scale. The average enzyme concentration was 250 U/L and the concentration of BPA in the influent was 100 µg/L. The operation conditions are as follows: temperature 25°C; real wastewater (pH 7.41); stirring, 150 rpm; 10 cycles of 24 h for each one.

The disappearance of BPA is determined by gas chromatography coupled to mass spectrometry. The enzyme activity was determined spectrophotometrically. The results show that the system is able to eliminate a high percentage (93%) of BPA after 10 cycles. (Figure 4).

## Claims

1. A method for eliminating organic microcontaminants present in secondary effluents from wastewater treatment plants (WWTP) or industrial effluents comprising a sequencing batch reactor:
a. applying a pre-treatment to the effluent that reduces the concentration of suspended solids and colloids in the effluent;
b. pumping the effluent towards a reaction vessel enzyme containing an enzyme with magnetic supports that immobilise the enzyme;
c. a reaction of the effluent with the immobilised enzyme;
d. retaining the immobilised enzyme by applying an external magnetic field;
e. discharging the stream that is free of microcontaminants to the aquatic environment;
f. eliminating the external magnetic field; and
g. pumping a fresh effluent to the reaction vessel.

2. The method according to claim 1, **characterised in that** the pre-treatment comprises a filtration treatment.

3. The method according to claims 1 and 2, **characterised in that** the pre-treatment comprises the use of a microfiltration membrane or a sand filter with a grain size of less than 1mm.

4. The method according to claim 1, **characterised in that** the immobilised enzyme is selected from peroxidases or laccases.

5. The method according to claim 4, **characterised in that** the activity of the immobilised peroxidase enzyme is comprised in the range of 50-1000 UL⁻¹.

6. The method according to claim 4, **characterised in that** the activity of the immobilised laccase enzyme is comprised in the range of 100-1500 UL⁻¹.

7. The method according to claims 1 and 4 to 6, **characterised in that** the operating temperature of the vessel is comprised in the range of 10 to 40°C.

8. The method according to claims 1 and 4 to 6, **characterised in that** the pH of the effluent mixture and the immobilised enzyme in the reaction vessel is comprised in the range of 3 to 8.

9. The method according to claims 1 and 4 to 8, **characterised in that** the reaction of the effluent further comprises the following stages of enzymatic activity control:
a. measuring enzymatic activity in the reaction vessel;
b. if the enzymatic activity in the vessel is less than a minimum value, the supports will be regenerated and the enzyme will be immobilised again for its addition;

10. The method, according to any one of the claims 1 and 4 to 9, **characterised in that** if the enzyme used is a peroxidase-type enzyme, hydrogen peroxide is continuously added to the reaction vessel.

11. The method according to claim 10, **characterised in that** hydrogen peroxide is added at a flow rate comprised between 5 and 1000 µmol/L·min.

12. The method according to claims 1 and 4 to 11, **characterised in that** if the enzyme used is manganese peroxidase (MnP) or versatile peroxidase (VP), dicarboxylic organic acid is also continuously added.

13. The method according to claim 12, **characterised in that** the dicarboxylic organic acid is added at a flow rate comprised between 1 and 100 µmol/L·min.

14. The method according to claims 1 and 4 to 11, **characterised in that** the necessary cofactor for completing the catalytic cycle of the peroxidase is also added to the reaction vessel.

15. The method according to claim 14, **characterised in that** the cofactor comprises veratryl alcohol if the enzyme used is a lignin peroxidase (LiP).

16. The method according to claim 15, **characterised in that** veratryl alcohol is introduced at a flow rate comprised in the range of 0.5-1000 µmol/L·min.

17. The method, according to claim 14, **characterised in that** the cofactor comprises Mn²⁺ in the case that the enzyme used is a peroxidase of the MnP type or of the VP type.

18. The method according to claim 17, **characterised in that** Mn²⁺ is introduced at a flow rate comprised in the range of 0.5 and 100 µmol/L·min.

19. The method according to any one of claims 1 to 9, **characterised in that** it also comprises measuring the concentration of dissolved oxygen in the reaction vessel when the enzyme used is a laccase-type enzyme.

20. The method, according to any of the preceding claims, **characterised in that** the concentration of microcontaminants is measured in the effluent inlet and in the outlet of the reactor.

21. The procedure according to claim 20, **characterised in that** if the concentration of contaminants in the outlet stream is greater than a fixed maximum value, the reaction time of the effluent with the enzyme immobilised is increased and the outlet recirculates to the inlet stream to be treated again.

22. The method according to claim 1, **characterised in that** the supports comprise silica-coated magnetic nanoparticles.

23. The method according to claim 22, **characterised in that** the nanoparticles have a size comprised in the range of 4-40 nm.

24. The method according to claim 22 **characterised in that** the silica coating is comprised in the range of 0.5-10 nm.

25. The method according to claims 22 and 23, **characterised in that** the magnetic nanoparticles comprise magnetic iron oxide (magnetite or maghemite) with a diameter preferably comprised in the range of 3 to 20 nm and have superparamagnetic properties.

26. The method according to claim 1, **characterised in that** the reaction comprises stirring the effluent with the mixture of the immobilised enzyme and magnetic nanoparticles.

27. A system for eliminating organic microcontaminants present in secondary effluents from wastewater treatment plants (WWTP) or industrial effluents comprising:
a. a pre-treatment system that reduces the concentration of suspended solids and colloids in the effluent;
b. a first pumping system;
c. a reaction vessel or reactor that comprises an enzyme solution with magnetic supports that immobilise the enzyme;
d. a stirring system;
e. an enzymatic activity control system for the solution contained in the reaction vessel;
f. a magnetic separation system;
g. a second pumping system;
h. a system for measuring microcontaminants in the enzymatic reaction vessel and in the outlet of the reactor.

28. The system according to claim 27, **characterised in that** the pre-treatment comprises a system of filtration treatment through a microfiltration membrane.

29. The system according to claim 27, **characterised in that** the pre-treatment comprises the use of a sand filter with a grain size of less than 1 mm.

30. The system according to claim 27, **characterised in that** the first pumping system pumps the effluent from the pre-treatment system to the reaction vessel.

31. The system according to claim 27, **characterised in that** the reaction vessel comprises a stirred-tank reactor.

32. The system, according to claim 31, **characterised in that** the stirring system comprises a four-blade stirrer, coated in Teflon to prevent problems of adsorption of nanoparticles and of compounds to be degraded.

33. The system according to claim 27, **characterised in that** the immobilised enzyme is selected from peroxidase or laccase.

34. The system according to claim 27, **characterised in that** the enzymatic activity control system comprises:
a. an enzymatic activity sensor; and
b. a system for the regeneration of supports and subsequent immobilisation of the enzyme.

35. The system according to claims 27 and 30 to 34, **characterised in that** the operating temperature of the vessel is comprised in the range of 10 to 40°C.

36. The system according to claims 27 and 30 to 34, **characterised in that** the pH of the effluent mixture and the immobilised enzyme in the reaction vessel is comprised in the range of 3 to 8.

37. The system, according to any one of the claims 27 and 30 to 36, **characterised in that** if the enzyme used is a peroxidase-type enzyme, it also comprises a system of continuously adding hydrogen peroxide to the reaction vessel.

38. The system according to claim 37, **characterised in that** the system for adding hydrogen peroxide adds hydrogen peroxide at a flow rate comprised between 5 and 1000 µmol/L·min.

39. The system, according to claim 37, **characterised in that** reaction vessel further comprises a system of continuously adding a dicarboxylic organic acid when the enzyme used is MnP or VP.

40. The system according to claim 39, **characterised in that** the system for adding a dicarboxylic organic acid adds the dicarboxylic organic acid at a flow rate comprised between the range of 1 and 100 µmol/L·min.

41. The system, according to claim 27, **characterised in that** the reaction vessel further comprises a system of adding the necessary cofactor for completing the catalytic cycle of the peroxidase.

42. The system according to claim 41, **characterised in that** the cofactor comprises veratryl alcohol if the enzyme used is a peroxidase of the LiP type.

43. The system according to claim 42, **characterised in that** the system of adding the cofactor adds veratryl alcohol at a flow rate comprised in the range of 0.5-1000 µmol/L·min.

44. The system according to claim 41, **characterised in that** the cofactor comprises Mn²⁺ if the enzyme used is an MnP- or VP-type peroxidase.

45. The system according to claim 44, **characterised in that** the system of adding the cofactor adds Mn²⁺ at a flow rate comprised in the range of 0.5-100 µmol/L·min.

46. The system according to claims 27 and 30 to 45, **characterised in that** the reaction vessel further comprises a control system for dissolved oxygen in the enzyme-effluent mixture in the case that the enzyme used is laccase, which comprises a sensor for measuring the concentration of dissolved oxygen.

47. The system according to claim 27, **characterised by** an external magnetic separation system.

48. The system according to claim 47, **characterised in that** the external magnetic separation system comprises one or several magnetic rods formed by permanent magnets aligned and mounted with alternate polarities, which are introduced during the retention phase in non-magnetic sheaths placed in the reaction vessel.

49. The system according to claim 27, **characterised in that** the second pumping system pumps the stream free of microcontaminants after the enzymatic treatment in the reactor.

50. The system according to claim 27, **characterised in that** if the concentration of contaminants in the outlet stream is greater than a fixed maximum value, the enzymatic activity control system increases the reaction time of the effluent with the immobilised enzyme and recirculates the outlet to the inlet stream to be treated again.

51. The system according to claim 27, **characterised in that** the supports comprise silica-coated magnetic nanoparticles.

52. The method according to claim 51, **characterised in that** the nanoparticles have a size comprised in the range of 4-40 nm.

53. The method according to claim 51 **characterised in that** the silica coating is comprised in the range of 0.5-10 nm.

54. The system according to claims 51 and 52, **characterised in that** the magnetic nanoparticles comprise magnetic iron oxide (magnetite or maghemite) with a diameter preferably comprised in the range of 3 to 20 nm and have superparamagnetic properties.

55. A use of the method according to claims 1 to 26, and of the system, according to claims 27 to 54, for eliminating organic microcontaminants present in secondary effluents from wastewater treatment plants (WWTP) or industrial effluents.
